(19) Europäisches Patentamt
European Patent Office
Office européen des brevets

(11) EP 1 465 640 B1

(12) EUROPÄISCHE PATENTSCHRIFT

(45) Veröffentlichungstag und Bekanntmachung des Hinweises auf die Patenterteilung:
**17.09.2008 Patentblatt 2008/38**

(21) Anmeldenummer: **03729214.1**

(22) Anmeldetag: **10.01.2003**

(51) Int Cl.:
***A61K 31/715*** (2006.01) ***A61K 31/716*** (2006.01)
***A61K 31/717*** (2006.01) ***A61K 31/718*** (2006.01)
***A61K 31/719*** (2006.01) ***A61K 31/721*** (2006.01)
***A61P 31/04*** (2006.01) ***A61P 31/12*** (2006.01)

(86) Internationale Anmeldenummer:
**PCT/DE2003/000095**

(87) Internationale Veröffentlichungsnummer:
**WO 2003/057227 (17.07.2003 Gazette 2003/29)**

# (54) VERWENDUNG VON POLYSACCHARID-DERIVATEN ALS ANTIINFEKTIVE SUBSTANZEN

USE OF POLYSACCHARIDE DERIVATIVES AS ANTI-INFECTIVE SUBSTANCES

UTILISATION DE DERIVES DE POLYSACCHARIDE COMME SUBSTANCES ANTI-INFECTIEUSES

(84) Benannte Vertragsstaaten:
**AT BE BG CH CY CZ DE DK EE ES FI FR GB GR HU IE IT LI LU MC NL PT SE SI SK TR**

(30) Priorität: **10.01.2002 DE 10200717**

(43) Veröffentlichungstag der Anmeldung:
**13.10.2004 Patentblatt 2004/42**

(73) Patentinhaber:
- **Dow Wolff Cellulosics GmbH
29699 Bomlitz (DE)**
- **Friedrich-Schiller-Universität Jena
07743 Jena (DE)**

(72) Erfinder:
- **HAACK, Vera
07743 Jena (DE)**
- **HEINZE, Thomas
07743 Jena (DE)**
- **SCHMIDTKE, Michaela
07743 Jena (DE)**
- **MÖLLMANN, Ute
07749 Jena (DE)**
- **DAHSE, Hans-Martin
99423 Weimar (DE)**
- **HÄRTL, Albert
07743 Jena (DE)**

(74) Vertreter: **Polypatent
Postfach 40 02 43
51410 Bergisch Gladbach (DE)**

(56) Entgegenhaltungen:
**EP-A- 0 948 960** **WO-A-00/26447**
**WO-A-98/29099** **US-A- 4 708 951**
**US-A- 5 254 540** **US-A- 5 595 980**
**US-B1- 6 306 835**

- **DATABASE WPI Section Ch, Week 199216 Derwent Publications Ltd., London, GB; Class A14, AN 1992-126185 XP002242820 & JP 04 057969 A (UNITIKA LTD), 25. Februar 1992 (1992-02-25)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft die Verwendung von Substanzen auf der Basis von Oligo- und Polysacchariden als antiinfektive, wie antibakterielle und antivirale Mittel. Diese antiinfektiven Mittel können beispielsweise in kosmetischen und pharmazeutischen Formulierungen als Konservierungsstoffe, als biologische aktive Verbindung in Arzneimittelzubereitungen, zur bioziden Ausrüstung von Oberflächen, von Geweben, und von Verpackungsmaterialien für beispielsweise Lebensmittel oder Produkte, die in der Medizin, Biologie, Pharmazie Verwendung finden, und für die Wundabdeckung zur Anwendung in der kosmetischen und pharmazeutischen Industrie, der Landwirtschaft und der Lebensmittel- und Futtermittelindustrie genutzt werden.

Es ist bekannt, dass global die Infektionen mit bakteriellen Krankheitserregern zunehmen und dabei die antibakterielle Resistenz ein generelles Gesundheitsproblem ist. Ein weltweiter Zuwachs an Tuberkuloseinfektionen mit Mykobakterienstämmen, die im Verlauf der Zeit resistent gegenüber den gewöhnlichen Therapeutika geworden sind (B.R.Bloom, J.L.Murray, Science 257, 1992, 1055), sowie die Therapie von Infektionen mit multiresistenten Staphylokokken (M. Kresken, Bundesgesundheitsblatt 38,1996, 170), erfordert das Design von neuen Wirksubstanzen. Alternative Wirkstoffe mit neuen Wirkmechanismen, insbesondere zur Entgegenwirkung der Antibiotikaresistenz und zur Bekämpfung bakterieller Infektionen bei Unverträglichkeiten gegenüber vorhandenen Wirkstoffen sind dringend notwendig.

Mit der Entwicklung hochselektiver Nukleosid- und Nukleotidvirustatika wie z. B. Acyclovir, Penciclovir, Ganciclovir, Sorivudine und Cidofovir für Herpesviren wurde zwar ein bedeutender Fortschritt bei der Bekämpfung lebensbedrohlicher Infektionen erreicht, doch die genannten Therapeutika besitzen alle das gleiche Wirkprinzip. Sie hemmen die virale DNA-Polymerase. Ein weiterer Nachteil dieser Verbindungen besteht darin, dass sie auch in den DNA-Stoffwechsel der infizierten Zelle eingreifen und deshalb die Gefahr in sich bergen, mutagene, teratogene und onkogene Wirkungen hervorzurufen (Wutzler, P. Thust, R. Antiv. Res. 49, 2001, 55). Außerdem wurden bei Langzeitanwendung von Nukleosid- und Nukleotidvirustatika sowohl in infizierten Zellkulturen als auch bei immunsuppremierten Patienten Resistenzentwicklungen gegenüber diesen Medikamenten nachgewiesen (Andrei, G. et al. Antimicrob. Agents Chemother., 1995, 39 1632; Pavic, I. et al. Antimicrob. Agents Chemother., 1997, 39 2686). Deshalb müssen zusätzlich neue hochwirksame antivirale Prophylaktika und Therapeutika mit einem anderen Wirkmechanismus entwickelt werden.

Quartäre Ammoniumverbindungen gehören zur großen Gruppe der biologisch wirksamen Substanzen. Sie sind in der Lage, Mikroorganismen wie Bakterien und Pilze zu vernichten. Niedermolekulare quartäre Ammoniumsalze werden als Desinfektionsmittel oder biocides Beschichtungsmaterial eingesetzt (J. Controlled Release 50, 1998, 145). Ein typisches Problem der niedermolekularen Verbindungen ist eine unzureichende Bioverfügbarkeit beispielsweise hervorgerufen durch verschiedene Transport- und Abbauvorgänge. Polymere quartäre Aminfunktionalisiertee Materialien können aus kommerziellen quartären Austauscherharzen, durch Graftpolymerisation von Polyurethanen mit Hydroxytelechelen des Polybutadienes oder aus Polysiloxanen mit primären Alkoholfunktionen in der Seitenkette synthetisiert werden. Diese bioziden Polymere haben meist hohe Produktionskosten und sind häufig toxisch, da Reste der toxischen Monomere enthalten sind (Trends in Polymer Science 4, 1996, 364). Darüber hinaus kann es zu unerwünschten und gefährlichen Akkumulationen der Polymere im Organismus kommen, da sie nicht biologisch abbaubar sind. Des weiteren werden synthetische Polymere, die kationische Funktionen enthalten, als Dispersionen für den Holzschutz verwendet (US 5,049,383). Nachteil der synthetischen Polymere, die kationische Funktionen enthalten, sind die hohen Herstellungskosten, die Toxizität (Verunreinigung durch Restmonomergehalt) und die Stabilität gegenüber biologischem Abbau.

Polysaccharidderivate mit quartären Ammoniumfunktionen sind bekannt und werden bisher vor allem als Zusatzstoff zur Oberflächenvergütung für die Papier- und Textilindustrie sowie in der Kosmetik als Konsistenzregler eingesetzt, wobei sie nur einen geringen Substitutionsgrad (DS) $< 0,2$ aufweisen. Über die biologischen Wirkungen ist bisher nichts bekannt geworden. Demgegenüber werden antiinfektive Wirkungen, speziell antibakterielle Wirkungen von Stärkeethern beschrieben, die lange Alkylketten ($C_8$-$C_{22}$) enthalten und an die Stärke über Silylethergruppen gebunden sind (JP 05295002). Die geringe chemische Stabilität der Alkylsilylether von Polysacchariden führt zu einer unkontrollierten Freisetzung von funktionellen Gruppen sogar schon durch die Einwirkung von Luftfeuchtigkeit und damit zur Verringerung oder zum Verlust der biologischen Aktivität (D. Klemm et.al., Comprehensive Cellulose Chemistry, Wiley-VCH, 1998). Darüber hinaus sind niedermolekuare Silylverbindungen toxisch. Ansonsten beziehen sich Veröffentlichungen auf antibakteriell wirksame Cellulosefasern und Chitosanderivate (W. H. Daly, M. M. Guerrini, Polym. Mat. Sci. Eng. 79, 1998, 220). Chitosan wird als natürliches kationisches Polysaccharid am häufigsten beschrieben und als fungizides Mittel in der Kosmetik eingesetzt (T. Tashiro, Macromol. Mater. Eng. 286, 2001, 63, K.C. Gupta, M.N.V.R. Kumar, J.M.S.-Rev. Macromol. Chem. Phys. C40, 2000, 273). Nachteile dieser Polysaccharide sind häufig die Verunreinigungen mit anderen biogenen Substanzen, der hohe Preis aufgrund der aufwendigen Isolierungs- und Reinigungsmethoden und die naturgegebene Struktur, die Ammoniumgruppen sind ausschließlich am Polymerrückgrat lokalisiert. Darüber hinaus ist deren Verteilung nicht steuerbar und der Gehalt ist auf einen Substitutionsgrad von 1 beschränkt. Superabsorber aus kationisch modifizierten und vernetzten Polysacchariden wie der Cellulose (EP 0 582 624 B1) wurden ebenfalls beschrieben.

US-A-5 254 540 beschreibt ammoniumfunktionelle, antimikrobielle Stärkederivate auf Pyridin-, Anilin-, Pyrazin- oder Alkylen-Basis, wobei ein Teil des Stärkegerüstes zu Aldehydfunktionen oxidiert ist.

Es existieren Angaben in der Literatur, dass die biologische Aktivität aus dem Vorhandensein der quartären Ammoniumfunktionen resultiert, wobei andererseits beschrieben wird, dass bei typischen Verbindungen mit kationischen Tetraalkylstickstoffgruppen wie Polyquaternium 10 nachweislich keine Bioaktivität vorliegt (W.A. Daly, M.M. Guerrini, D. Culberson, J. Macossay, in: Science and Technology of Polymers and Advanced Materials, Plenum Press 1998, 493). Es lässt sich aus den vorliegenden Literaturergebnissen keineswegs schlussfolgern, ob und welche Strukturen tatsächlich biologisch wirksam sind.

Der Erfindung liegt die Aufgabe zugrunde, Polymere mit neuartiger antiinfektiver Wirksamkeit aufzufinden; die Substanzen sollen eine hohe antiinfektive Wirkung in einem breiten Spektrum besitzen, eine effektive Bekämpfung von

Antibiotikaresistenz bei bakteriellen Infektionen gestatten, neue Möglichkeiten zur Therapie von viralen Infektionen bieten, sowie gut verträglich, biologisch abbaubar, nicht toxisch und in einfacher Art herstellbar sind.

Die Aufgabe wird erfindungsgemäß durch die Bereitstellung von Polysaccharidderivaten laut Anspruch 1 gelöst, bei denen durch Veretherungsreaktionen kationische Funktionen mit einem Substitutionsgrad (DS) im Bereich von 0,4 bis 3,0, insbesondere Alkylammoniumgruppierungen mit einem DS im Bereich von 0,6 bis 1,8 über Spacergruppen in Polysaccharide eingeführt wurden. Polysaccharide laut Anspruch 1 sind aufgrund ihrer biologischen Abbaubarkeit und Nichttoxizität besonders geeignet.

Die Erfindung betrifft demgemäß die Verwendung von Polysacchariden laut Anspruch 1, die mit über Linker gebundenen quaternären Ammoniumgruppen mit einem Substitutionsgrad von 0,4 bis 3,0 substituiert sind, als antiinfektive Mittel bzw. zur Behandlung von Infektionserkrankungen.

Die erfindungsgemäß verwendeten Verbindungen laut Anspruch 1 weisen eine hohe biologische Aktivität auf und hemmen überraschenderweise das Wachstum pathogener Bakterien, wie z.B. Staphylokokken und Mycobakterien mit minimalen Hemmkonzentrationen im Bereich von 5 - 60 mg/L sowie die Vermehrung von Herpes- und Influenzaviren in einem Bereich von 3-50 mg/L. Die Verbindungen können auf Grund dieser Eigenschaften zur Herstellung von Arzneimitteln zur Vorbeugung und Bekämpfung bakterieller und viraler Infektionen dienen. Sie können sowohl allein als auch in Kombination mit bekannten Therapeutika oder mit physiologisch verträglichen Hilfs- und Trägerstoffen angewandt werden.

Die antiinfektiven Verbindungen laut Anspruch 1 können zur Verwendung als Lösung oder Suspension in pharmazeutisch akzeptablen Medien für eine topische oder parenterale Applikation, über intravenöse, subcutane oder intramuskuläre Injektionen, für intranasale Applikation; als Tablette, Kapsel oder Suppositorium zubereitet werden. Die Verbindungen können in Dosierungen von 0,1 - 1000 mg/kg Körpergewicht eingesetzt werden.

Zur Herstellung der erfindungsgemäßen wirksamen Verbindungen werden bevorzugt Stärken wie native Stärken unterschiedlicher Provenienz, beispielsweise Kartoffel-, Weizen-, Mais- und Reisstärke, und chemisch oder enzymatisch partiell hydrolysierte Stärken wie Solamyl, Amylose, Amylopektin und Wachsmaisstärke sowie aus gentechnisch veränderten Pflanzen gewonnene Stärken wie die Hylon-Typen eingesetzt. Der Gehalt an Amylose in den Stärken kann ebenso wie der an Amylopektin jeweils von 0-100%, bevorzugt von 30-70%, betragen. Die Molekulargewichte geeigneter Polysaccharide liegen im Bereich von $10^3$-$10^7$g/mol (vgl. Tab. 1). Die Anhydroglucoseeinheit-Wiederholungseinheiten (AGU) können über $\alpha$(1-4), ($\alpha$(1-6), $\alpha$(1-3), $\beta$(1-4) und $\beta$(1-3) Bindungen oder auch Kombinationen davon wie beispielsweise $\alpha$(1-4) und $\alpha$(1-6), wie in Abb. 1 schematisch gezeigt, oder $\alpha$(1-6), $\alpha$(1-3) und $\alpha$(1-4) miteinander verknüpft sein und unterschiedlich lange und verknüpfte Seitenketten enthalten. Darüber hinaus können auch weitere funktionelle Gruppen wie Phosphatesterfunktionen beispielsweise bei der natürlichen Kartoffelstärke enthalten sein.

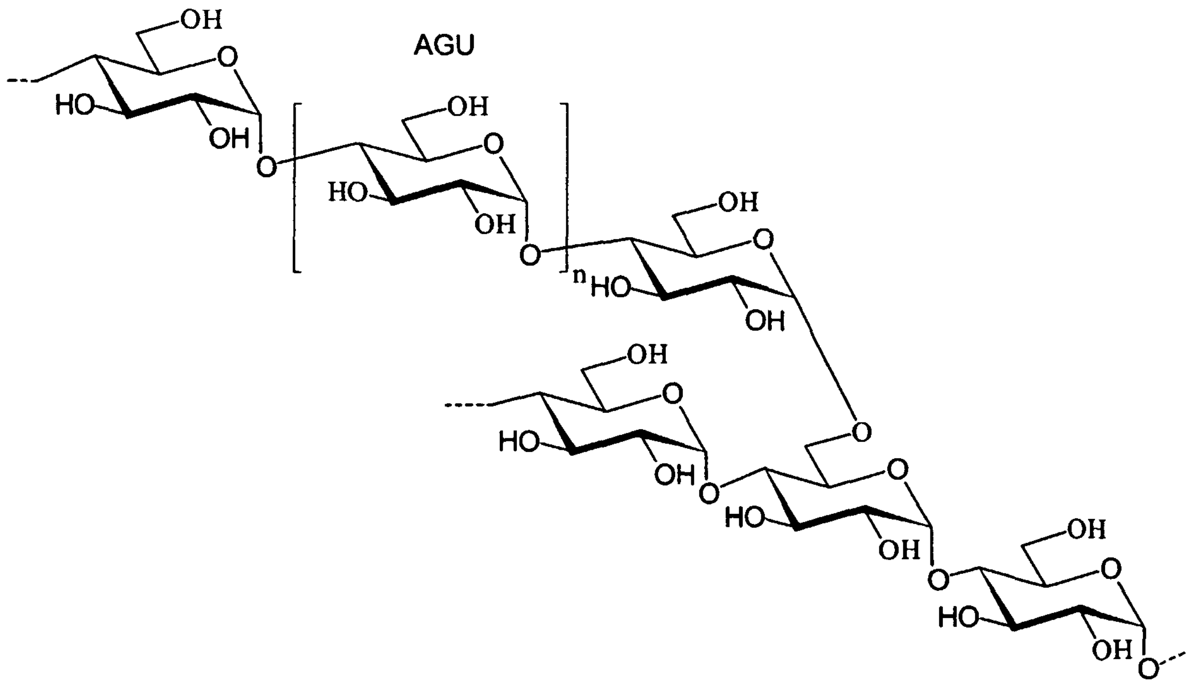

Abb. 1 Strukturbeispiel der einsetzbaren Polysaccharide

Tab. 1:

| Stärkematerial | Amylosegehalt (%) | Molekulargewicht (GPC[1]) ($g \cdot mol^{-1}$) |
|---|---|---|
| Hylon VII (**H**) | 70 | $9 \cdot 10^6$ |
| Kartoffelstärke (**P**, Emsland) | 28 | $40 \cdot 10^6$ |
| Maisstärke (**M**) | 28 | $76 \cdot 10^6$ |
| Weizenstärke (**W**) | 26 | $65 \cdot 10^6$ |
| Wachsmaisstärke (**WM**) | 1 | $51 \cdot 10^6$ |
| Solamyl (**S**) | 28 | 9700 |

[1]bestimmt in DMSO

Das Ausmaß der Umsetzung an den Hydroxylgruppen wird durch den durchschnittlichen Substitutionsgrad (DS) beschrieben. Dieser Durchschnittswert gibt ohne jede Differenzierung die Anzahl der funktionalisierten Hydroxylgruppen an und liegt demnach bei den genannten Polysacchariden definitionsgemäß im Bereich von 0 bis 3. Der DS an kationischen Gruppen der antiinfektiv wirksamen Polysaccharidderivate der Erfindung liegt zwischen 0,4 und 3,0, bevorzugt zwischen 0,6 und 1,8. Werden bei Derivatisierungen funktionelle Gruppen eingeführt, die selbst reaktive Gruppierungen enthalten, z.B. Hydroxylgruppen als Ergebnis der Veretherung von Polysacchariden mit Epoxiden, können diese unter Ausbildung von längeren Seitenketten ebenfalls reagieren.

Die erfindungsgemäß verwendbaren Polysaccharidderivate sind bekannt oder können in an sich bekannter Weise gewonnen werden, insbesondere durch Veretherung von Polysacchariden mit reaktiven Verbindungen, wodurch entweder direkt quartäre Ammoniumverbindungen der allgemeinen Formel (I) (PS: Polysaccharid-Rest, nur ein Substituent gezeigt) gebildet werden oder wobei die Quaternisierung im Anschluss an die Veretherungsreaktion erfolgt. In Formel (!) bedeutet $R_1$, $R_2$ und $R_3$ unabhängig voneinander vorzugsweise Alkyl mit 1-4 C Atomen oder Benzyl oder substituiertes Benzyl (Substituenten sind z.B. 1 bis 3 Alkyl, Halogen, Alkoxy, Carbamoyl, Alkoxycarbonyl, Cyano, Dialkylamino), $R_1$ auch Wasserstoff, X ein Anion (z.B. Halogenid, Hydroxid, Sulfat, Hydrogensulfat und ein anderes Anion von Mineral- und Carbonsäuren), n 2-4 sein kann.

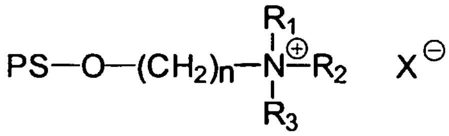

(I)

Bevorzugt verwendete quartäre Kationisierungsreagenzien sind das 2,3-Epoxypropyltrimethylammoniumchlorid (QUAB®151, Degussa AG, Formel II) oder das 3-Chloro-2-hydroxypropyltrimethylammoniumchlorid (QUAB®188, Degussa AG, Formel III). Es lassen sich auch Reagenzien der allgemeinen Formel IV mit Y = Cl, Br und n = 1-3 zur Veretherung der Polysaccharide heranziehen.

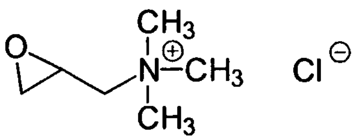

(II)

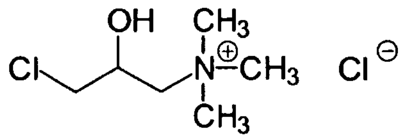

(III)

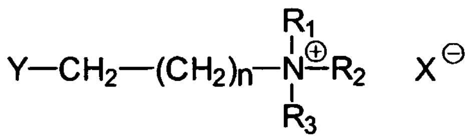

(IV)

Demgemäß handelt es sich bei dem Linker, über den die quaternären Ammoniumgruppen an die Polysaccharide gebunden sind, um gegebenenfalls durch Hydroxy substituiertes $C_2$-$C_4$-Alkylen.

Die Veretherung zur Herstellung der biologisch aktiven Polysaccharidderivate kann in unterschiedlicher Weise durchgeführt werden und erfolgt in an sich bekannter Weise, wobei hohe Gehalte an den kationischen Gruppen realisiert werden. Sowohl Suspensionen der Polymere in einem Alkohol und Natronlauge und Wasser (heterogenes Verfahren), wobei Alkohole wie Methanol, Isopropanol und bevorzugt Ethanol, oder wässrigen Alkalilaugen, bevorzugt Natronlauge/Wasser mit einem Übergang vom heterogenen zum homogenen System als auch homogene Lösungen der Polymere in dipolar-aprotischen Lösemitteln wie Dimethylsulfoxid oder Dimethylacetamid in Gegenwart von Lithiumchlorid oder weiteren Lösemitteln als Reaktionsmedien geeignet sind. Die Reaktionszeit für die Umsetzung mit dem Kationisierungsreagenz liegt zwischen 1 und 48 h, bevorzugt bei 3 bis 24 h, und die Temperatur zwischen 30 und 130°C, bevorzugt bei 40 bis 80°C. Durch die eingesetzten Moläquivalente des Veretherungsmittels kann zusätzlich der Substitutionsgrad der Produkte eingestellt und in weiten Grenzen variiert werden. Darüber hinaus sind auch Mehrstufenreaktionen für die Gewinnung der Polysaccharidderivate geeignet, wobei ein bereits kationisiertes oder aminofunktionalisiertes Produkt erneut unter den oben genannten Bedingungen umgesetzt wird. Die Aufarbeitung der Reaktionsprodukte erfolgt mit üblichen Verfahren der Polymerchemie, wobei die niedermolekularen Nebenprodukte und Reagenzreste durch Dialyse oder Waschprozesse oder Umfällen aus Wasser in organische Lösemittel abgetrennt werden.

Die Substitutionsgrade werden mittels Elementaranalyse ermittelten Stickstoffwerten nach folgender Bestimmungsformel berechnet:

$$DS_N = \frac{162{,}15 \cdot \%N}{1401 - 151{,}64 \cdot \%N}$$

Darüber hinaus eignen sich die in den Verbindungen enthaltenen Gegenionen wie Chlorid und die NMR-Spektren zur Bestimmung der DS-Werte.
Die folgenden Ausführungsbeispiele sollen die Erfindung näher erläutern, jedoch in keiner Weise einschränken.

Ausführungsbeispiele:

1. Herstellung der Verbindungen durch heterogene Reaktionsführung in Ethanol/Natronlauge/Wasser

20 g Polysaccharid (Art des Polysaccharides, siehe folgende Tabelle 2) werden in 80 ml Ethanol suspendiert. Zu dieser Suspension wird eine Lösung von 10,85 g NaOH in 28 ml Wasser und 80 ml Ethanol sowie eine Lösung von 0,246 mol QUAB®188 (69% wässrige Lösung) zugetropft. Das Reaktionsgemisch wird für 6 h bei 60°C gerührt. Das Produkt wird mit 0,1 n HCl neutralisiert, dialysiert und gefriergetrocknet.
Die Ausbeute beträgt 95 % (bezogen auf den erreichten DS).
Elementaranalyse: N % 3,51
Durchschnittlicher Substitutionsgrad ($DS_N$) = 0,66

Tab. 2: Heterogene Kationisierung

| Reagenz | | | | Substitutionsgrad |
|---|---|---|---|---|
| Polysaccharid | Typ | Molverhältnis AGU : Reagenz | Produkt | |
| Hylon | QUAB®188 | 1 : 3 | H 1 (466) | 0,50 |
| Hylon | QUAB®188 | 1 : 2 | H 2 (KS005) | 0,66 |
| Amioca | QUAB®188 | 1 : 3 | WM 1 (505) | 0,14 |
| Kartoffelstärke | QUAB®188 | 1 : 3 | P 1[1] (491) | 0,34 |
| Kartoffelstärke | QUAB®188 | 1 : 3 | P 2' (469) | 0,58 |
| Weizenstärke | QUAB®188 | 1 : 3,25 | W 1 (527) | 0,99 |
| Weizenstärke | QUAB®188 | 1 : 2 | W 3 (571) | 0,61 |
| Weizenstärke | 2 | 1 : 5 | W 4 | 0,72 |
| Weizenstärke | 2 | 1 . 10 | W 5 | 0,81 |

[1] unterschiedliche NaOH-Konzentration
[2] Reagenz: $Cl\text{-}CH_2\text{-}CH_2\text{-}N(C_2H_5)H_2Cl$

2. Umsetzung von Polysacchariden in wässriger Natronlauge

20 g Polysaccharid (Art des Polysaccharides, siehe folgende Tabelle 3) werden in einer Natronlaugelösung (0,5 g NaOH in 100 ml Wasser) suspendiert. Das Gemisch wird für eine Stunde bei 60°C gerührt. Bei dieser Temperatur werden 0,123 mol QUAB®151 bzw. im Fall der abgebauten Stärke Solamyl wird QUAB® 188 zugetropft. Das Reaktionsgemisch wird für 6 h bei 60°C gerührt. Nach Abkühlung auf Raumtemperatur wird das Gemisch mit 0,1 n HCl neutralisiert; anschließend dialysiert und gefriergetrocknet.
Die Ausbeute beträgt 98% (bezogen auf den erreichten DS).
Elementaranalyse: N % 3,34
Durchschnittlicher Substitutionsgrad ($DS_N$) = 0,60

Tab. 3: Hetero-/homogene Kationisierung

| Reagenz | | | | Substitutionsgrad |
|---|---|---|---|---|
| Polysaccharid | Typ | Molverhältnis AGU : Reagenz | Produkt | |
| Hylon | QUAB®151 | 1 : 1 | H 3 (477) | 0,40 |
| Hylon | QUAB®151 | 1 : 2 | H 4 (KS006) | 0,92 |
| Amioca | QUAB®151 | 1 : 0,5 | WM 2 (503) | 0,38 |
| Amioca | QUAB®151 | 1 : 1 | WM 3 (501) | 0,60 |

(fortgesetzt)

| Reagenz | | | | Substitutionsgrad |
|---|---|---|---|---|
| Polysaccharid | Typ | Molverhältnis AGU : Reagenz | Produkt | |
| Amioca | QUAB®151 | 1 : 2 | WM 4 (502) | 0,92 |
| Maisstärke | QUAB®151 | 1 : 0,5 | M 1 (517) | 0,35 |
| Maisstärke | QUAB®151 | 1 : 1 | M 2[1] (516) | 0,55 |
| Maisstärke | QUAB®151 | 1 : 1 | M 3[1] (519) | 0,72 |
| Maisstärke | QUAB®151 | 1 : 2 | M 4 (515) | 1,03 |
| Kartoffel | QUAB®151 | 1 : 3 | P 3 (KS 016) | 0,69 |
| Kartoffel | QUAB®151 | 1 : 2 | P 4 (KS 013) | 1,05 |
| Weizenstärke | QUAB®151 | 1 : 0,5 | W 4 (520) | 0,39 |
| Solamyl | QUAB®188 | 1 : 2 | S 1[1] (554) | 0,68 |
| Solamyl | QUAB®188 | 1 : 3 | S 2 (555) | 0,76 |
| Solamyl | QUAB®188 | 1 : 2 | S 3[1] (568) | 0,80 |

[1] unterschiedliche NaOH-Konzentration

3. Homogene Reaktionsführung in Dimethylsulfoxid (DMSO)

15 g Polysaccharid (Art des Polysaccharides, siehe folgende Tabelle 4) werden in Dimethylsulfoxid (DMSO) bei Raumtemperatur suspendiert und auf 80°C erwärmt, wobei sich das Polysaccharid auflöst. Die Lösung wird auf Raumtemperatur abgekühlt und 0,5 g NaOH gelöst in 20 ml Wasser zugesetzt. Anschließend werden 0,0925 mol QUAB®151 unter Rühren zugetropft. Das Reaktionsgemisch wird für 24 h bei 60°C gerührt. Nach Abkühlung auf Raumtemperatur wird das Gemisch mit 0,1 n HCl neutralisiert, anschließend dialysiert und gefriergetrocknet.
Die Ausbeute beträgt 99% (bezogen auf den erreichten DS).
Elementaranalyse: N % 3,22
Durchschnittlicher Substitutionsgrad ($DS_N$) = 0,57

Tab. 4: Homogene Kationisierung

| Reagenz | | | | Substitutionsgrad |
|---|---|---|---|---|
| Polysaccharid | Typ | Molverhältnis AGU : Reagenz | Produkt | |
| Hylon | QUAB®151 | 1 : 3 | H 5 (436) | 0,55 |
| Kartoffelstärke | QUAB®151 | 1 : 1 | P 5 (KS 9) | 0,42 |
| Amioca | QUAB®151 | 1 : 1 | WM 5 (504) | 0,57 |
| Weizenstärke | QUAB®151 | 1 : 1 | W 5 (531) | 0,41 |
| Maisstärke | QUAB®151 | 1 : 1 | M 5 (532) | 0,40 |
| Solamyl | QUAB®151 | 1 : 1 | S 4 (588) | 0,60 |

4. Kationisierung in mehreren Stufen

5 g kationisiertes Polysaccharid (Art des Polysaccharides, siehe folgende Tabelle 5) werden in einer Natronlaugelösung (0,5 g NaOH in 100 ml Wasser) suspendiert. Das Gemisch wird für eine Stunde bei 60°C gerührt. Bei dieser Temperatur werden 0,2 mol QUAB®151 zugetropft. Das Reaktionsgemisch wird für 6 h bei 60°C gerührt. Nach Abkühlung auf Raumtemperatur wird das Gemisch mit 0,1 n HCl neutralisiert; anschließend dialysiert und gefriergetrocknet.

Die Ausbeute beträgt 95% (bezogen auf den erreichten DS).
Elementaranalyse: N % 5,11
Durchschnittlicher Substitutionsgrad ($DS_N$) = 1,32

Tab. 5: Kationisierung in mehreren Stufen

| Kationisierte Stärke | | Reagenz | | | Substit. Grad |
|---|---|---|---|---|---|
| Polysaccharid | Ausgangs-DS | Typ | Molverh. AGU:Reag. | Produkt | $DS_N$ |
| Hylon | 0,40 | QUAB®151 | 1 : 2 | H 6 (479) | 0,90 |
| Hylon | 0,80 | QUAB®151 | 1 : 1,5 | H 7 (498) | 1,10 |
| Kartoffel | 0,77 | QUAB®151 | 1 : 2 | P 6 (506) | 1,18 |
| Kartoffel | 0,42 | QUAB®151 | 1 : 10 | P 7 (597) | 1,32 |
| Amioca | 0,60 | QUAB®151 | 1 : 10 | WM 6 (598) | 1,25 |
| Weizen | 0,41 | QUAB®151 | 1 : 10 | W 6 (603) | 1,16 |
| Weizen | 0,88 | QUAB®151 | 1 : 10 | W 7 (602) | 1,41 |
| Mais | 0,55 | QUAB®151 | 1 : 10 | M 6 (600) | 1,13 |
| Kartoffel | 1,32 | QUAB®151 | 1 : 15 | P 8 | 1,80 |
| Mais | 1,13 | QUAB®151 | 1 : 10 | M 7 | 1,65 |
| Solamyl | 0,80 | QUAB®151 | 1 : 2 | S 5 (610) | 0,91 |

5. Bestimmung der antibakteriellen/antimikrobiellen Aktivität der Verbindungen gegen grampositive und gramnegative Bakterien und gegen Hefen.

Die antibakterielle Aktivität der Verbindungen gegen Staphylococcus aureus SG 511, S. aureus 134/93 (multiresistent) und Mycobacterium vaccae IMET 10670 wurde mittels Bestimmung der minimalen Hemmkonzentrationen (MHK) in einem Mikro-Bouillon-Verdünnungstest in Müller-Hinton Bouillon (DIFCO) entsprechend der NCCLS-Richtlinien [National Committee for Clinical Laboratory Standards: Methods for dilution antimicrobial susceptibility tests for bacteria that grow aerobically; 4th Ed.; Villanova, Ed.; Approved standard Document M7-A4. NCCLS, (1997)] geprüft. Die Ergebnisse sind in Tabelle 6 dargestellt.

Tab. 6: Antibakterielle Aktivität

| Probe | Substitutions-grad $DS_N$ | MHK [mg/L] Staphylococcus aureus SG 511 | MHK [mg/L] Staphylococcus aureus 134/93 | MHK [mg/L] Mycobacterium vaccae IMET10670 |
|---|---|---|---|---|
| H 4 KS006 | 0,92 | 15,6 | 15,6 | 7,8 |
| H 7 498 | 1,10 | 15,6 | 15,6 | 7,8 |
| WM 4 502 | 0,92 | 31,25 | 62,5 | 15,6 |
| M 3 519 | 0,72 | 15,6 | 62,5 | 7,8 |
| M 4 515 | 1,03 | 31,25 | 62,5 | 15,6 |
| P 3 016 | 0,69 | 31,25 | 62,5 | 7,8 |
| P 4 013 | 1,05 | 15,6 | 125 | 3,9 |
| W 2 567 | 0,57 | 62,5 | 62,5 | 15,6 |
| W 3 571 | 0,61 | 31,25 | 31,25 | 7,8 |
| W 1527 | 0,99 | 31,25 | 31,25 | 15,6 |
| S 1 554 | 0,68 | 31,25 | 62,6 | 15,6 |
| S 2 555 | 0,76 | 15,6 | 31,25 | 7,8 |
| S 3 568 | 0,80 | 15,6 | 15,6 | 7,8 |

6. Bestimmung der antiviralen Wirkung gegenüber Herpes simplex Virus Typ 1

Vor den antiviralen Untersuchungen wurde die 50 %ig zytotoxische Konzentration ($CC_{50}$) in Green monkey kidney (GMK) Zellen bestimmt, um unspezifische Substanzwirkungen ausschließen zu können. Dazu werden geschlossene GMK-Zellrasen in Mikrotiterplatten mit den entsprechenden Substanzverdünnungs-reihen (Faktor 2) beimpft (Schmidtke et al.; J. Virol. Meth. 95, 2001, 133). Nach einer 72-stündigen Inkubation erfolgt die Färbung der Zellen mit Kristallviolett/Methanol. Nach dem Herauslösen des Farbstoffes wird die optische Dichte der einzelnen Vertiefungen in einem Plattenphotometer der Firma Dynatech (550/630 nm) gemessen und mit dem Mittelwert von 6 unbehandelten Zellkontrollen verglichen, der als 100 % angenommen wird. Die $CC_{50}$ stellt die Substanzkonzentration im Schnittpunkt der Extinktionskurve der Verdünnungsreihe mit der 50 % Linie des Kontrollmittelwertes dar. Die antivirale Wirkung der Verbindungen gegenüber HSV-1 wurde im zytopathischen Effekt-Hemmtest (zpE-Hemmtest) in GMK-Zellen untersucht und die 50 %ige Hemmdosis ($IC_{50}$) ermittelt (Schmidtke et al.; J. Virol. Meth. 95, 2001, 133). Der Selektionsindex wurde als Quotient aus $CC_{50}$ und $IC_{50}$ berechnet (Tabelle 7).
Die Ausgangsverbindungen (QUAB-Reagenzien bzw. nichtmodifizierte Stärken) zeigten keine antivirale Wirkung (Ergebnisse nicht dargestellt).

Tab. 7: Antivirale Aktivität

| Probe | Substitutions-grad $DS_N$ | $CC_{50}$ (µg/ml) in GMK-Zellen | $IC_{50}$ (µg/ml) gegenüber HSV-1 | Selektionsindex ($CC_{50}/IC_{50}$) |
|---|---|---|---|---|
| **H 3** 477 | 0,40 | >200 | 8,54 | > 23,42 |
| **H 1** 466 | 0,50 | >200 | 5,11 | > 39,14 |
| **H 2** 005 | 0,66 | > 200 | 7,07 | > 28,29 |
| **WM 2** 503 | 0,38 | > 200 | 7,05 | > 28,37 |
| **M 1** 517 | 0,35 | > 200 | 7,84 | > 25,51 |
| **P 1** 491 | 0,34 | > 200 | 10,15 | > 19,70 |
| **W 4** 520 | 0,39 | > 200 | 10,55 | > 18,96 |
| **S 1** 554 | 0,68 | 141,54 | 3,59 | 39,43 |

7. Bestimmung der Wirkungsweise im modifizierten PRT

Die Untersuchungen zum Wirkmechanismus der Substanz wurden mit dem Acyclovir- und Phosphonoameisensäure-sensitivem HSV1 Stamm Kupka in einem modifiziertem PRT beispielhaft mit der Verbindung M 1 durchgeführt (Schmidtke et al.; J. Virol. Meth. 95, 2001, 133). Hierbei erfolgte die Substanzzugabe in verschiedenen Konzentrationen:

1. nur zu zellfreiem Virus ($10^6$ pfu/ml), welches dann mit der Verbindung für 6h bei 37°C inkubiert und nach Ausverdünnen der Substanz in den PRT einbezogen wurde: keine Plaquereduktion im Dosisbereich bis 6,25-25 µg/ml (Ergebnisse nicht dargestellt),
2. nur im Agar: keine Plaquereduktion im Dosisbereich 6,25-25 µg/ml (Ergebnisse nicht dargestellt),
3. nur während der einstündigen Adsorption für 2 h bei 4°C (3,12-12,5 µg/ml) und
4. 1, 2 und 4 h vor Viruszugabe (3,12-12,5 µg/ml).

Die Ergebnisse der Untersuchungen zum Wirkmechanismus zeigen, dass die erfindungsgemäßen Verbindungen nicht viruzid wirken, denn eine Inaktivierung von zellfreiem Virus konnte nicht festgestellt werden. Voraussetzung für die Hemmung der Herpesvirusreplikation ist vielmehr die Anwesenheit der Substanz vor bzw. während der Virusadsorption an die Testzellen.

## Patentansprüche

1. Verwendung von alpha-glycosidisch verknüpften Stärke-Polysaccharid-Derivaten der allgemeinen Formel (I)

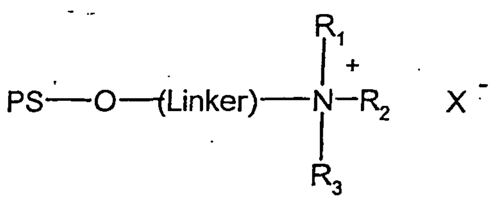

wobei

PS für einen Polysaccharid-Rest auf Stärke-Basis steht,
X ein Anion bedeutet,
$R^1$ Wasserstoff, $C_1$-$C_4$-Alkyl oder Benzyl oder substituiertes Benzyl bedeutet,
$R^2$ und $R^3$ unabhängig voneinander jeweils $C_1$-$C_4$-Alkyl oder Benzyl oder substituiertes Benzyl bedeuten und

Linker eine gegebenenfalls durch Hydroxy substituierte $C_2$-$C_4$-Alkylen-Gruppe ist,
die mit den über Linker gebundenen quaternären Ammoniumgruppen bei einem Substitutionsgrad von 0,4 bis 3,0 substituiert sind,
zur Herstellung eines Medikamentes gegen Infektionskrankheiten.

2. Verwendung gemäß Anspruch 1, wobei der Substitutionsgrad 0,6 bis 1,8 beträgt.

3. Verwendung gemäß Anspruch 1, wobei es sich bei den Polysacchariden um Stärken wie Kartoffen-, Weizen-, Mais- und Reisstärke, chemisch oder enzymatisch partiell hydrolysierte Stärke oder aus gentechnisch veränderten Pflanzen gewonnene Stärke handelt.

## Claims

1. Use of alpha-glycosidically linked starch-polysaccharide derivatives of the general formula (I)

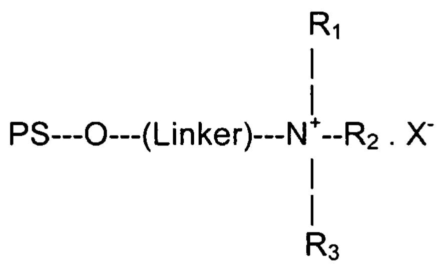

where

PS signifies a polysaccharide residue based on starch,
X signifies an anion,
$R^1$ signifies hydrogen, $C_1$-$C_4$ alkyl or benzyl or substituted benzyl,
$R^2$ and $R^3$ signify, independently of one another, $C_1$-$C_4$ alkyl or benzyl or substituted benzyl, and

linker is a $C_2$-$C_4$ alkylene group, possibly substituted by hydroxy,
the quaternary ammonium groups bound via linkers are substituted with a degree of substitution of 0.4 to 3.0,
for the production of a medicine against infectious diseases.

2. Use according to Claim 1, the degree of substitution being 0.6 to 1.8.

3. Use according to Claim 1, the polysaccharides being starches such as potato, wheat, maize and rice starches, chemically or enzymatically partly hydrolysed starch or starch obtained from genetically modified plants.

## Revendications

1. Emploi de dérivés de polysaccharides d'amidon à liaisons alpha-glycosidiques, de formule générale (I) :

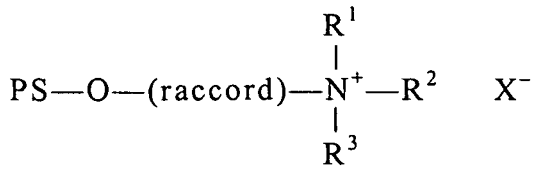

dans laquelle
PS représente un reste de polysaccharide à base d'amidon,
X représente un anion,
$R^1$ représente un atome d'hydrogène, un groupe alkyle en $C_{1-4}$, un groupe benzyle, ou un groupe benzyle à substituant(s),
$R^2$ et $R^3$ représentent chacun, indépendamment l'un de l'autre, un groupe alkyle en $C_{1-4}$, un groupe benzyle, ou un groupe benzyle à substituant(s),
et le "raccord" est constitué d'un groupe alkylène en $C_{2-4}$, qui peut éventuellement porter un ou plusieurs substituant (s) hydroxy,
lesquels dérivés comportent les substituants que sont les groupes de type ammonium quaternaire liés par l'intermédiaire du raccord en un degré de substitution qui vaut de 0,4 à 3,0,
en vue de la fabrication d'un médicament contre des maladies infectieuses.

**2.** Emploi conforme à la revendication 1, pour lequel ledit degré de substitution vaut de 0,6 à 1,8.

**3.** Emploi conforme à la revendication 1, pour lequel les polysaccharides sont des amidons comme les amidons de pomme de terre, de blé, de maïs ou de riz, des amidons partiellement hydrolysés par voie chimique ou enzymatique, ou des amidons obtenus à partir de plantes génétiquement modifiées.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5049383 A
- JP 05295002 B
- EP 0582624 B1
- US 5254540 A


### In der Beschreibung aufgeführte Nicht-Patentliteratur


- **B.R.BLOOM ; J.L.MURRAY.** *Science,* 1992, vol. 257, 1055
- **M. KRESKEN.** *Bundesgesundheitsblatt,* 1996, vol. 38, 170
- **WUTZLER, P. ; THUST, R.** *Antiv. Res.,* 2001, vol. 49, 55
- **ANDREI, G. et al.** *Antimicrob. Agents Chemother.,* 1995, vol. 39, 1632
- **PAVIC, I. et al.** *Antimicrob. Agents Chemother.,* 1997, vol. 39, 2686
- *J. Controlled Release,* 1998, vol. 50, 145
- *Trends in Polymer Science,* 1996, vol. 4, 364
- **D. KLEMM.** Comprehensive Cellulose Chemistry. Wiley-VCH, 1998
- **W. H. DALY ; M. M. GUERRINI.** *Polym. Mat. Sci. Eng.,* 1998, vol. 79, 220
- **T. TASHIRO.** *Macromol. Mater. Eng.,* 2001, vol. 286, 63
- **K.C. GUPTA ; M.N.V.R. KUMAR.** *J.M.S.-Rev. Macromol. Chem. Phys.,* 2000, vol. C40, 273
- **W.A. DALY ; M.M. GUERRINI ; D. CULBERSON ; J. MACOSSAY.** Science and Technology of Polymers and Advanced Materials. Plenum Press, 1998, 493
- **SCHMIDTKE et al.** *J. Virol. Meth.,* 2001, vol. 95, 133